# EUROPÄISCHE PATENTSCHRIFT

(11) **EP 1 127 133 B1**
(45) Veröffentlichungstag und Bekanntmachung des Hinweises auf die Patenterteilung: **26.01.2005**
(21) Anmeldenummer: 99963187.2
(22) Anmeldetag: 03.11.1999
(51) Int. Cl.: C12N 15/36, C07K 14/02, C12N 15/62, C07K 19/00, C07K 16/08, C12N 15/87

(54) **ZELLPERMEABILITÄT-VERMITTELNDES POLYPEPTID**
POLYPEPTIDE MEDIATING CELL PERMEABILITY
POLYPEPTIDE CONFERANT UNE PERMEABILITE CELLULAIRE

(30) Priorität: 03.11.1998 DE 19850718
(43) Veröffentlichungstag der Anmeldung: 29.08.2001
(73) Patentinhaber: Island Pharmaceuticals Ltd., IM1 2BB British Isles (GB)
(72) Erfinder: HILDT, Eberhard, 81675 München (DE); SCHMIDT, Stephanie, 81675 München (DE)
(74) Vertreter: Vossius, Volker, Dr.
(86) Internationale Anmeldenummer: PCT/DE1999/003506
(87) Internationale Veröffentlichungsnummer: WO 2000/026379

(56) Entgegenhaltungen:
- EP-A- 0 456 215
- WO-A-95/20657
- HILDT E. ET AL.: "Characterization of essential domains for the functionality of the MHBst trabscriptional activator and identification of a minimal MHBst activator" ONCOGENE, Bd. 11, Nr. 10, 16. November 1995 (1995-11-16), Seiten 2055-2066, XP000922823
- WIEPRECHT T. ET AL.: "Influence of the angle subtended by the positively charged helix face on the membrane activity of amphipathic, antibacterial peptides" BIOCHEMISTRY, Bd. 36, 1997, Seiten 12869-12880, XP002141072
- OESS S. UND HILDT E. : "Novel cell permeable motif derived from the PreS2-domain of hepatitis-B virus surface antigens" GENE THERAPY, Bd. 7, Nr. 9, Mai 2000 (2000-05), Seiten 750-758, XP000922825
- LEHNINGER: 'BIOCHEMIE', Bd. 3, 2001, SPRINGER VERLAG Seite 123
- SAMBROOK; FRITSCH; MANIATIS: 'MOLECULAR CLONING - a laboratory manual. 2nd edition. Seiten 9.47-9.62, 11.46', 1989, COLD SPRING HARBOUR LABORATORY PRESS
- BOLTON E.T.; MCCARTHY B.J.: 'A general method for the isolation of RNA complementary to DNA' PROCEEDINGS OF THE NATIONAL ACADEMY OF SCIENCES OF USA Bd. 48, 1962, Seiten 1390 - 1397
- TOSHINORI OZAKI ET AL: 'Interaction of DA41, a DAN-binding protein, with the Epidermal Growth Factor-like protein, S(1-5)' BIOCHEMICAL AND BIOPHYSICAL RESEARCH COMMUNICATIONS Bd. 237, Nr. 2, 18 August 1997, Seiten 245 - 250
- SHUM B.P. ET AL: 'A divergent non-classical class I gene conserved in salmonids' IMMUNOGENETICS Bd. 49, Nr. 6, April 1999, Seiten 479 - 490

## Beschreibung

Die vorliegende Erfindung betrifft ein Polypeptid, das zellpermeabel ist und Zell-permeabilität an Substanzen vermitteln kann, und eine für ein solches Polypeptid kodierende DNA. Ferner betrifft die Erfindung die Verwendung des Polypeptids zur Vermittlung von Zellpermeabilität an Substanzen.

Mit Zellpermeabilität wird die Eigenschaft von Substanzen bezeichnet, in Zellen einzudringen. Diese Eigenschaft findet sich allerdings nur bei wenigen Substanzen. Die meisten Substanzen benötigen Hilfsmittel bzw. Verfahren, um in Zellen einzudringen. Beispiele hierfür sind Mikroinjektion, Elektroporation, Assoziation mit kationischen Lipiden, Liposomenbildung, Rezeptor-vermittelte Endocytose und Virusinfektion. Diese Hilfsmittel bzw. Verfahren weisen jedoch große Nachteile auf. Insbesondere sind sie teuer, erfordern komplexe Versuchsanordnungen und sind nur begrenzt einsetzbar. Femer ist ihre Effizienz gering und sie erweisen sich oft als toxisch.

Der vorliegenden Erfindung liegt somit die Aufgabe zugrunde, ein Mittel bereitzustellen, mit dem Substanzen in Zellen eingebracht werden können, wobei vorstehende Nachteile vermieden werden.

Erfindungsgemäß wird dies durch die Gegenstände in den Patentansprüchen erreicht.

Das Dokument "HILDT E. et al.: 'Characterization of essential domains for the functionality of the MHBs' transcriptional activator and identification of a minimal MHBs' activator' ONCOGENE, Bd. 11, Nr. 10, 16. November 1995, Seiten 2055-2066" offenbart ein Peptid, das die Aminosäuresequenz " Pro-Ile-Ser-Ser-Ile-Phe-Ser-Arg-Thr-Gly-Asp-Pro " aufweist (D4: Abbildung 7).

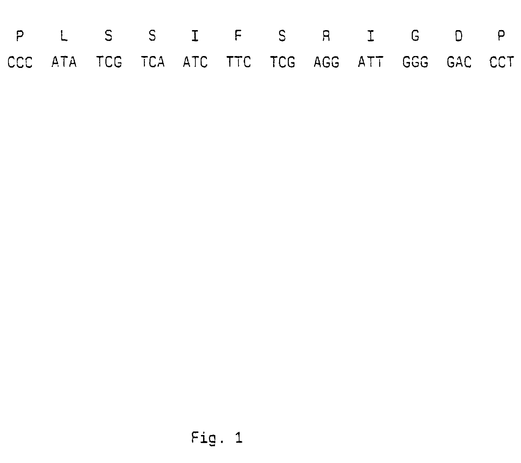

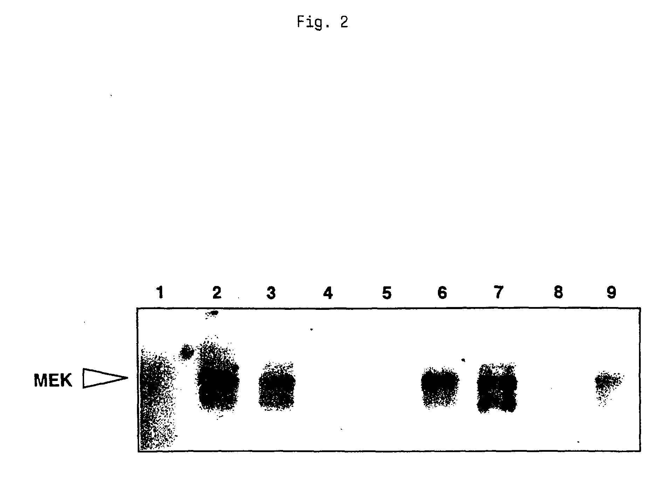

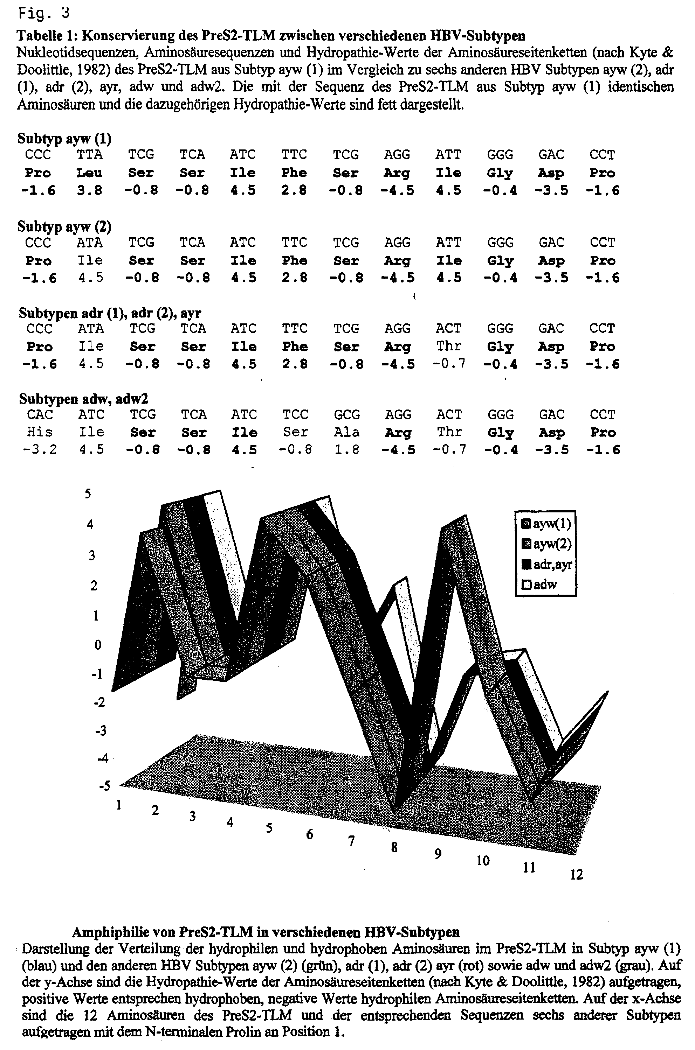

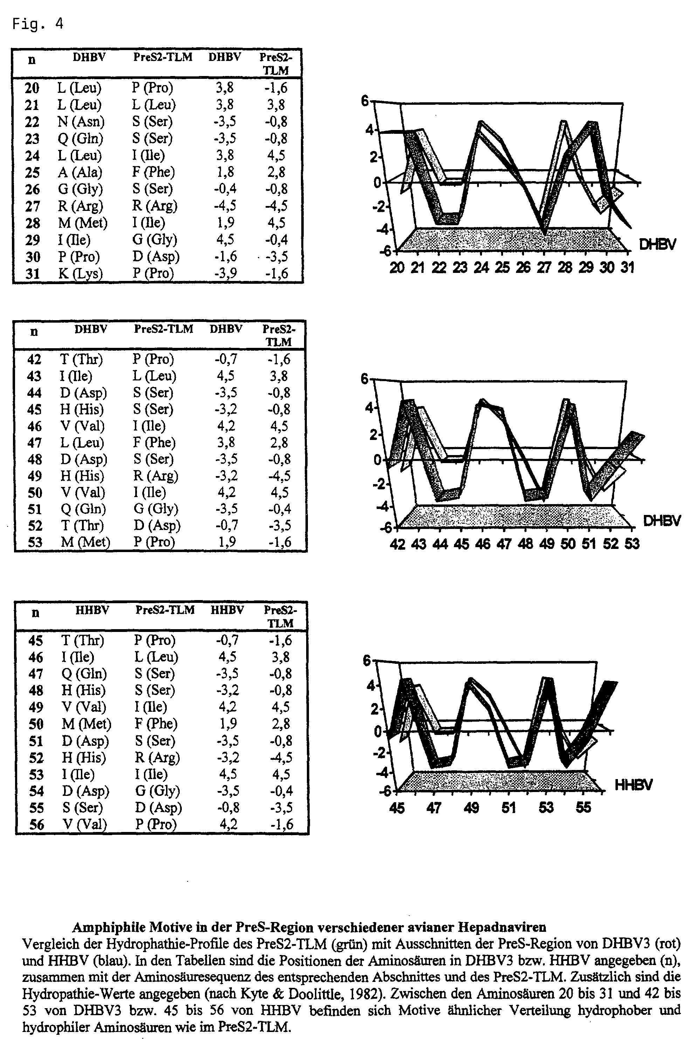

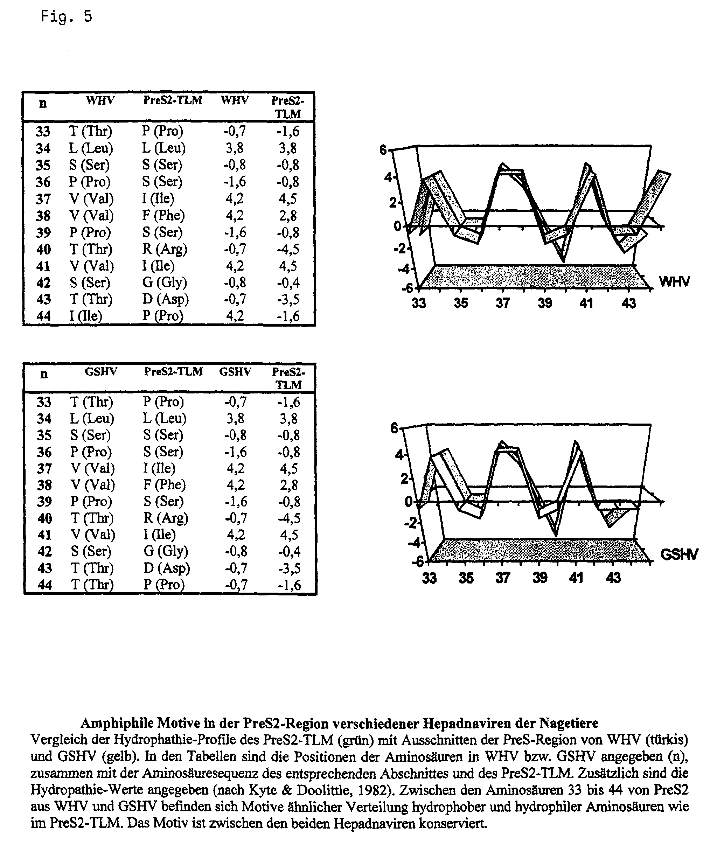

Die vorliegende Erfindung beruht auf den Erkenntnissen des Anmelders, dass ein Polypeptid, das vorzugsweise die Aminosäuresequenz von Fig. 1 umfasst, in Zellen eindringen kann, d.h. Zellpermeabilität aufweist. Er hat ein solches Polypeptid in der PreS2-Region eines Hepatitis B Virus (HBV)-Oberflächenproteins gefunden. Das Polypeptid wird nachstehend mit ZPP "Zellpermeabilität-vermittelndes Polypeptid" bezeichnet. ZPP weist die Struktur einer amphiphilen α-Helix auf. Ferner hat der Anmelder erkannt, dass ZPP die Eigenschaft hat, Zellpermeabilität an Substanzen zu vermitteln. Diese können dann in Zellen eindringen, wobei die Zellpermeabilität der Substanzen nicht auf bestimmte Zellen beschränkt ist. Auch behalten die Substanzen ihre Aktivitäten bei (vgl. Fig. 2). Varianten des erfindungsgemäßen ZPP aus verschiedenen HBV-Subtypen, die sich von der Sequenz von Fig. 1 durch eine oder mehrere Aminosäuren unterscheiden, sind in Fig. 3 gezeigt. Der Aminosäure-Sequenz von Fig. 1 (= PreS2-TLM) entspricht beispielsweise der Subtyp ayw (1) auf der Aminosäureebene vollständig, während die anderen Subtypen demgegenüber einen oder mehrere Austausche aufweisen. Man sieht aber, dass zwischen verschiedenen HBV-Subtypen eine Konservierung bestimmter Aminosäuren vorhanden ist. Diese lässt sich insbesondere durch die graphische Darstellung der Hydrophobizitätswerte bestätigen. Hieraus kann man den Schluss ziehen, dass selbst bei einem Austausch von einer oder mehreren Aminosäuren, die Hydropathie-Verteilung im Gesamtmolekül erhalten bleiben sollte. Aufgrund dieses Befunds ist es für den Fachmann leicht möglich, Varianten der Sequenz von Fig. 1 zu bestimmen, da nicht die Sequenz als solche entscheidend ist, sondern das Hydropathieprofil im Gesamtmolekül. Entsprechendes gilt für verschiedene aviane Hepadnaviren (vgl. Fig. 4) bzw. Hepadnaviren der Nagetiere (vgl. Fig. 5). Diese werden jeweils mit dem erfindungsgemäßen ZPP gemäß Fig. 1 (= PreS2-TLM) verglichen und Hydropathie-Profile aufgestellt. Aus diesen ist ersichtlich, dass selbst bei einem fast vollständigen Austausch der Aminosäuren (z.B. HHBV <--> PreS2-TLM) das Hydropathie-Profil im Wesentlichen erhalten bleibt. Dies bedeutet, dass nicht die Sequenz an sich, sondern die Abfolge hydrophiler und hydrophober Aminosäuren in einem α-helikalen Motiv entscheidend ist.

Erfindungsgemäß werden die Erkenntnisse des Anmelders genutzt, ein Polypeptid (ZPP) bereitzustellen, das Zellpermeabilität an Substanzen vermitteln kann, wobei ZPP eine Aminosäuresequenz, die ausgewählt ist aus der Gruppe bestehend aus umfasst und über kovalente oder nicht-kovalente Bindungen mit einem in eine Zelle zu transportierenden Molekül verbunden ist, mit der Maßgabe, dass das ZPP kein natives HBV Oberflächenprotein ist.

Ferner kann erfindungsgemäß ein Zellpermeabilität-vermittelndes Polypeptid (ZPP) in vitro dazu verwendet werden, Zellpermeabilität an eine in eine Zelle zu transportierende Substanz zu vermitteln, wobei das ZPP eine Aminosäuresequenz, ausgewählt aus der Gruppe bestehend aus umfasst und über kovalente oder nicht-kovalente Bindungen mit der Substanz verbunden wird.

Der Ausdruck "Vermittlung von Zellpermeabilität an Substanzen" weist darauf hin, dass ZPP Zellpermeabilität an Substanzen jeglicher Art und Abstammung vermitteln kann. Die Substanzen können z.B. Polypeptide (Proteine), Nukleinsäuren oder chemische Verbindungen sein. Beispiele von Polypeptiden sind Struktur-Polypeptide, Tumornekrosefaktor, Interferone, Interleukine, Lymphokine, Wachstumsfaktoren, Plasmaproteine, z.B. Gerinnungsfaktoren und Stoffwechselenzyme, und Rezeptoren. Insbesondere können die Polypeptide solche sein, welche die Immunogenität von Zellen steigern können. Dies können Polypeptide sein, die Tumorzellen fehlen, z.B. Zytokine, wie IL-2, und GM-CSF, und kostimulatorische Moleküle, wie B7-1, tumorassoziierte Antigene, z.B. MAGE1, Tyrosinasen und virale Polypeptide, z.B. E7 von humanem Papillomvirus und EBNA-3-Polypeptid von Epstein-Barr-Virus. Femer können die Polypeptide Adapter-Polypeptide, Oligomerisierungsmotive eines Polypeptids, Polypeptidfragmente von Virus-Hüllpolypeptiden, Hormone und Ribozyme sein. Beispiele von Nukleinsäuren sind solche, die für vorstehende Polypeptide kodieren. Ferner können es Antisense-Oligonukleotide, Peptid-Nukleinsäuren und Consenus-Sequenzen für Transkriptionsfaktoren sein. Beispiele von chemischen Verbindungen sind Arzneimittel, die keine Polypeptid-Struktur aufweisen. Solche können Cytostatika, Anästhetika, Antihistaminika, Antibiotika und Antimycotika sein. Zur Vermittlung der Zellpermeabilität kann es ausreichend sein, wenn ZPP zusammen mit einer Substanz inkubiert wird, so dass sich chemische Bindungen, z.B. kovalente bzw. nicht-kovalente Bindungen, ausbilden können. Günstig ist es, wenn ZPP über einen Linker mit der Substanz verbunden ist, wobei dies z.B. über Biotin/Streptavidin erfolgen kann. Der Linker kann am N- oder C-Terminus von ZPP vorliegen. Besonders vorteilhaft ist es, wenn die Substanz als Polypeptid zusammen mit ZPP in einem Fusionspolypeptid vorliegt. ZPP kann hierbei am N- oder C-Terminus bzw. innerhalb der Polypeptid-Struktur der Substanz vorliegen. Der Ausdruck "ZPP" umfasst daher auch ein Fusionspolypeptid, in dem ZPP zusammen mit einer Substanz vorliegt. Die Vermittlung von Zellpermeabilität an Substanzen kann durch übliche Verfahren nachgewiesen werden. Günstig ist es, Zellen mit ZPP verbundenen Substanzen zu inkubieren und das Eindringen bzw. Vorliegen von ZPP und/oder den Substanzen in den Zellen nachzuweisen. Dies kann z.B. durch spezifische Antikörper oder Reagenzien erfolgen, die direkt oder indirekt mit ZPP bzw. den Substanzen reagieren.

Ein weiterer Gegenstand der vorliegenden Erfindung ist eine Nukleinsäure, die für ein ZPP darstellendes Polypeptid kodiert, das als Fusionspolypeptid mit einer in eine Zelle zu transportierenden Substanz vorliegt und eine Aminosäuresequenz umfasst, die aus den vorstehend beschriebenen Aminosäuresequenzen (II), (III) und (VI) bis (X) ausgewählt ist. Die Nukleinsäure kann eine RNA oder eine DNA sein, Bevorzugt ist eine DNA, die folgendes umfasst:
(a) Die DNA von Fig. 1 oder eine hiervon durch ein oder mehrere Basenpaare unterschiedliche DNA, wobei letztere DNA mit der DNA von Fig. 1 hybridisiert und nicht für ein natives HBV-Oberflächenprotein kodiert, oder
(b) eine mit der DNA von (a) über den degenerierten genetischen Code verwandte DNA.

Der Ausdruck "eine durch ein oder mehrere Basenpaare unterschiedliche DNA" umfasst jegliche für ein ZPP kodierende DNA-Sequenz, die mit der DNA von Fig. 1 hybridisiert und nicht für ein natives HBV-Oberflächenprotein kodiert. Die DNA-Sequenz kann sich von der DNA von Fig. 1 durch Additionen, Deletionen, Substitutionen und/oder Inversionen von ein oder mehreren Basenpaaren unterscheiden. Hinsichtlich des Ausdrucks "Hybridisierung" wird auf vorstehende Ausführungen entsprechend verwiesen.

Eine erfindungsgemäße DNA kann als solche oder in einem Vektor vorliegen. Insbesondere kann eine erfindungsgemäße DNA in einem Expressionsvektor vorliegen. Beispiele solcher sind dem Fachmann bekannt. Im Falle eines Expressionsvektors für E. coli sind dies z.B. pGEMEX, pUC-Derivate, pGEX-2T, pET3b und pQE-8. Für die Expression in Hefe sind z.B. pY100 und Ycpad1 zu nennen, während für die Expression in tierischen Zellen z.B. pKCR, pEFBOS, cDM8, pCEV4, pCDNA3, pKSV10, pRCMV und pRK5 anzugeben sind. Für die Expression in Insektenzellen eignet sich besonders der Bacculovirus-Expressionsvektor pAcSGHisNT-A.

Der Fachmann kennt geeignete Zellen, um die erfindungsgemäße, in einem Expressionsvektor vorliegende DNA zu exprimieren. Beispiele solcher Zellen umfassen die E.coli-Stämme HB101, DH1, x1776, JM101, JM 109, BL21, SG 13009 und M15pRep4, den Hefe-Stamm Saccharomyces cerevisiae, die tierischen Zellen L, NIH 3T3, FM3A, CHO, COS, Vero, HeLa, Hep62, CCL13 und 293, die Insektenzellen Sf9 und Sf21 und die Pflanzenzellen Lupinus albus.

Der Fachmann kennt Verfahren und Bedingungen, Zellen mit einem, die erfindungsgemäße DNA enthaltenden Expressionsvektor zu transformieren bzw. transfizieren und die Zellen zu kultivieren. Auch sind ihm Verfahren bekannt, das durch die erfindungsgemäße DNA exprimierte ZPP zu isolieren und zu reinigen.

Die vorliegende Erfindung ermöglicht es, Zellpermeabilität zu vermitteln. Mit einem erfindungsgemäßen ZPP kann Zellpermeabilität an Substanzen jeglicher Art und Abstammung vermittelt werden. Die Zellpermeabilität ist universell, d.h. sie ist nicht auf bestimmte Zellen beschränkt. Auch können die Zellen ex vivo vorliegen. Desweiteren löst die Zellpermeabilität keine toxischen Effekte aus.

Die vorliegende Erfindung eignet sich somit bestens für die Herstellung eines Arzneistoffes zur Diagnose und Therapie. Letzteres umfasst das Eingreifen in die Expression von Genen und in Stoffwechselprozesse. Besonders eignet sich die vorliegende Erfindung für die Herstellung eines Arzneistoffes zur Diagnose und Therapie schwerster Erkrankungen, z.B. von Tumoren. Ganz besonders zeichnet sich die vorliegende Erfindung dadurch aus, dass die Arzneistoffe sowohl für konservative als auch gentherapeutische Behandlungsmaßnahmen eingesetzt werden können.

Kurze Beschreibung der Zeichnungen.
- **Fig. 1**: zeigt die Aminosäure- und DNA-Sequenzen eines erfindungsgemäßen, Zellpermeabilität-vermittelnden Polypeptids (ZPP).
- **Fig. 2**: zeigt den Nachweis von durch ZPP vermittelter Zellpermeabilität. Die Spuren 2 und 3 zeigen die Aktivierung von c-Raf1-Kinase. Die Spuren 4 und 5 zeigen deren Hemmung. Die Spuren 6 und 7 zeigen, dass mutiertes ZPP-PLAP (ZPP-KLAP) keine Hemmwirkung aufweist.
- **Fig. 3**: zeigt die Konservierung der Aminosäuresequenz zwischen verschiedenen HBV-Subtypen sowie Hydropathieprofil
- **Fig. 4**: zeigt amphiphile Motive in der PreS-Region verschiedener avianer Hepadnaviren
- **Fig. 5**: zeigt amphiphile Motive in der PreS2-Region verschiedener Hepadnaviren der Nagetiere
- **Fig. 6**: zeigt, dass DHBV42-53-EGFP ein zellpermeables Protein ist

Die vorliegende Erfindung wird durch die nachstehenden Beispiele erläutert.

### Beispiel 1: Nachweis von durch ein erfindungsgemäßes Polypeptid (ZPP) vermittelter Zellpermeabilität.

Der Nachweis einer durch ZPP-vermittelten Zellpermeabilität wird durch Hemmung der TNFα-abhängigen Aktivierung von c-Raf1-Kinase gezeigt. Die Aktivierung der c-Raf1-Kinase beruht auf der Interaktion des TNF-Rezeptors I (TNF-RI) mit dem Adaptermolekül Grb2. Hierzu interagiert die SH3-Domäne von Grb2 mit einem PLAP-Motiv aus der cytoplasmatischen Domäne von TNF-RI.

Es wird ZPP in Form eines Fusionspolypeptids bereitgestellt. In diesem mit ZPP-PLAP bezeichneten Fusionspolypeptid liegt ZPP der Aminosäuresequenz von Fig. 1 als N-Terminuspartner und ein PLAP-Motiv aus der cytoplasmatischen Domäne von TNF-RI als C-Terminuspartner vor. Femer wird ein mit ZPP-KLAP bezeichnetes Fusionspolypeptid bereitgestellt, bei dem das PLAP-Motiv mutiert ist.

HeLa Zellen werden 2 h mit 2µM ZPP-PLAP bzw. ZPP-KLAP (Kontrolle) inkubiert und 15 min mit 100µ/ml TNFα stimuliert. Die Aktivierung von c-Raf1 Kinase wird durch einen Immunkomplex-Test unter Verwendung von MEK (Santa Cruz, Biotech) und γ³²P-ATP als Substrat bestimmt (vgl. Fig. 2).

Es zeigt sich, dass ZPP-PLAP in die Zellen gelangt und die Aktivierung von C-Raf1-Kinase vollständig hemmt (vgl. Fig. 2, Spuren 4 und 5). Ferner zeigt sich, dass ZPP-KLAP keine Hemmung erreicht (vgl. Fig. 2, Spuren 6 und 7).

### Beispiel 2: Herstellung und Reinigung eines erfindungsgemäßen Zellpermeabilltät-vermittelnden Polypeptids (ZPP).

Die DNA von Fig. 1 wird am 5'-Ende mit einem BgIII-Linker und am 3'-Ende mit einem BamHI-Linker versehen und mit den entsprechenden Restriktionsenzymen nachgespalten. Das erhaltene BgIII/BamHI-Fragment wird in den BamHI-gespaltenen Expressionsvektor pQe8 inseriert, so dass das Expressionsplasmid pQe8/ZPP erhalten wird.

Ferner wird aus dem Expressionsplasmid pGex-1 eine für GST (Glutathion-S-Transferase) kodierende Sequenz isoliert. Diese weist an ihrem 5'-Ende eine BamHI-Restriktionsschnittstelle gefolgt von einer für eine Thrombinschnittstelle kodierenden Sequenz auf. Ferner weist die Sequenz an ihrem 3'-Ende eine BamHI-Restriktionsschnittstelle auf. Die Sequenz wird in das BamHI-gespaltene Expressionsplasmid pQe8/ZPP inseriert, wodurch das Expressionsplasmid pQe8/ZPP-GST erhalten wird. Dieses kodiert für das Fusionspolypeptid ZPP-GST. pQe48/ZPP-GST wird zur Transformation von E.coli SG 13009 (vgl. Gottesmann, S. et al., J. Bacteriol. 148, (1981), 265-273) verwendet. Die Bakterien werden in einem LB-Medium mit 100µg/ml Ampicillin und 25µg/ml Kanamycin kultiviert und 4 h mit 60µM Isopropyl-β-D-Thiogalactopyranosid (IPTG) induziert. Nach Induktion wird mittels Ultraschall die Lyse der sedimentierten und gewaschenen Bakterien durchgeführt. Die Isolierung des ZPP-GST-Fusionspolypeptids erfolgt mittels Affinitätschromatographie an einer Glutathion-Säule. Das gebundene ZPP-GST-Fusionspolypeptid wird mittels eines linearen Anstiegs der Glutathion-Konzentration von 0 auf 10 mM eluiert. Das eluierte ZPP-GST Fusionsprotein wird einer Thrombinspaltung unterzogen: Das dadurch freigesetzte Hexa-His-ZPP (Fusionspolypeptid) wird anschließend durch Affinitätschromatographie unter denaturierenden Bedingungen mittels einer Ni-NTA-Agarose isoliert. Dies erfolgt in Gegenwart von 6 M Harnstoff entsprechend den Angaben des Herstellers (Qiagen). Das gebundene Hexa-His-ZPP wird in einem Puffer mit pH 6,3 eluiert, der 250 mM Imidazol enthält. Das Hexa-His-ZPP wird einer 18 %igen SDS-Polyacrylamid-Gelelektrophorese unterworfen und mit Coomassie-Blau angefärbt (vgl. Thomas, J.O. und Kornberg, R.D., J. Mol. Biol. 149 (1975), 709-733).

Es zeigt sich, dass ein erfindungsgemäßes (Fusions)polypeptid in hochreiner Form hergestellt werden kann.

### Beispiel 3: Herstellung und Nachweis eines Antikörpers

Ein erfindungsgemäßes Fusionspolypeptid von Beispiel 2 wird einer 18 % SDS-Polyacrylamid-Gelelektrophorese unterzogen. Nach Anfärbung des Gels mit 4 M Natriumacetat wird eine ca. 3 kD Bande aus dem Gel herausgeschnitten und in Phosphat gepufferter Kochsalzlösung inkubiert. Gel-Stücke werden sedimentiert, bevor die Proteinkonzentration des Überstandes durch eine SDS-Polyacrylamid-Gelelektrophorese, der eine Coomassie-Blau-Färbung folgt, bestimmt wird. Mit dem Gel-gereinigten Fusionspolypeptid werden Tiere wie folgt immunisiert:

### Immunisierungsprotokoll für polyklonale Antikörper im Kaninchen

Pro Immunisierung werden 35 µg Gel-gereinigtes Fusionspolypeptid in 0,7 ml PBS und 0,7 ml komplettem bzw. inkomplettem Freund's Adjuvans eingesetzt.
- Tag 0:: 1. Immunisierung (komplettes Freund's Adjuvans)
- Tag 14:: 2. Immunisierung (inkomplettes Freund's Adjuvans; icFA)
- Tag 28:: 3. Immunisierung (icFA)
- Tag 56:: 4. Immunisierung (icFA)
- Tag 80:: Ausbluten

Das Serum des Kaninchens wird im Immunoblot getestet. Hierzu wird ein erfindungsgemäßes Fusionspolypeptid von Beispiel 2 einer SDS-Polyacrylamid-Gelelektrophorese unterzogen und auf ein Nitrocellulosefilter übertragen (vgl. Khyse-Andersen, J., J. Biochem, Biophys. Meth. 10, (1984), 203-209). Die Westem Blot-Analyse wurde wie in Bock, C.-T. et al., Virus Genes 8, (1994), 215-229, beschrieben, durchgeführt. Hierzu wird das Nitrocellulosefilter eine Stunde bei 37°C mit einem ersten Antikörper inkubiert. Dieser Antikörper ist das Serum des Kaninchens (1:10000 in PBS). Nach mehreren Waschschritten mit PBS wird das Nitrocellulosefilter mit einem zweiten Antikörper inkubiert. Dieser Antikörper ist ein mit alkalischer Phosphatase gekoppelter monoklonaler Ziege-anti-Kaninchen-IgG-Antikörper (Dianova) (1:5000) in PBS. Nach 30-minütiger Inkubation bei 37°C folgen mehrere Waschschritte mit PBS und anschließend die alkalische Phosphatase-Nachweisreaktion mit Entwicklerlösung (36µM 5'-Bromo-4-chloro-3-indolylphosphat, 400µM Nitroblau-tetrazolium, 100mM Tris-HCl, pH 9.5, 100 mM NaCl, 5 mM MgCl₂) bei Raumtemperatur, bis Banden sichtbar werden.

Es zeigt sich, dass polyklonale Antikörper hergestellt werden können.

### Immunisierungsprotokoll für polyklonale Antikörper im Huhn

Pro Immunisierung werden 40µg Gel-gereinigtes Fusionspolypeptid in 0,8 ml PBS und 0,8 ml komplettem bzw. inkomplettem Freund's Adjuvans eingesetzt.
- Tag 0:: 1. Immunisierung (komplettes Freund's Adjuvans)
- Tag 28:: 2. Immunisierung (inkomplettes Freund's Adjuvans; icFA)
- Tag 50:: 3. Immunisierung (icFA)

Aus Eigelb werden Antikörper extrahiert und im Western Blot getestet. Es werden polyklonale Antikörper nachgewiesen.

### Immunisierungsprotokoll für monoklonale Antikörper der Maus

Pro Immunisierung werden 12µg Gel-gereinigtes Fusionspolypeptid in 0,25 ml PBS und 0,25 ml komplettem bzw. inkomplettem Freund's Adjuvans eingesetzt; bei der 4. Immunisierung ist das Fusionsprotein in 0,5 ml (ohne Adjuvans) gelöst.
- Tag 0:: 1, Immunisierung (komplettes Freund's Adjuvans)
- Tag 28:: 2. Immunisierung (inkomplettes Freund's Adjuvans; icFA)
- Tag 56:: 3. Immunisierung (icFA)
- Tag 84:: 4. Immunisierung (PBS)
- Tag 87:: Fusion

Überstände von Hybridomen werden im Western Blot getestet. Monoklonale Antikörper werden nachgewiesen.

### Beispiel 4: Nachweis der DHBV-ZPP vermittelten Zellpermeabilität

Nachweis der durch das DHBV(Duck Hepatitis B-Virus)-ZPP vermittelten Zellpermeabilität wurde folgendermaßen geführt. Es wurde gemäß Standardmethoden ein Fusionsprotein bestehend aus einem hexa-His-Tag (6H), dem DHBV-ZPP und eGFP (enhanced green fluorescent protein) analog Beispiel 1 in einem E. coli Expressionssystem hergestellt. Es wurde das pQE-Vektorsystem der Fa. Qiagen angewendet. Dieses Protein (DHBV42-53eGFP) wurde isoliert. Für Kontrollexperimente wurde wt6HeGFP (Fusionsprotein aus 6 His und eGFP) verwendet. Es wurden nun 293-Zellen für 10 und 20 Min. in Gegenwart dieser Proteine inkubiert. Die Proteine wurden in einer Konzentration von 1 µM dem Medium zugesetzt. Nach 10 bzw. 20 Min. wurden die Zellen lysiert und die cytosolische Fraktion der Zellen durch Ultrazentrifugation isoliert.

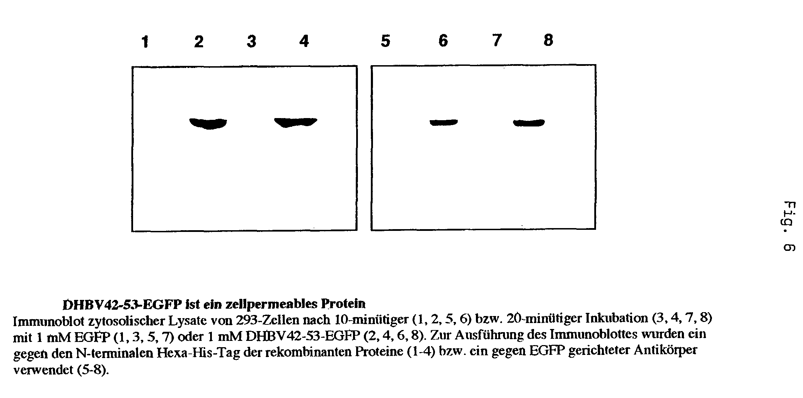

Der Nachweis des Vorhandenseins von DHBV42-53-eGFP in der Cytosolfraktion erfolgte mittels Western-Blot-Analyse unter Verwendung eines hexa-His-tag spezifischen Antikörpers (Fig. 6, Spuren 1-4) (anti-Hexa-His6 der Fa. Qiagen) bzw. eines eGFP-spezifischen Antikörpers (anti-eGFP der Fa. Clontech) (Fig. 6, Spuren 5-8). Zur Detektion wurde ein .Peroxidase-konjugierter Sekundärantikörper (anti-Maus HRP, anti-Rabbit-HRP der Fa. Amersham) verwendet.

Der Western Blot zeigt, dass im Falle der Zugabe von DHBV42-53-eGFP eine Intemalisation des Proteins in die Zelle (Cytosol) nach 10 Min. (Spuren 2, 6) bzw. 20 Min. (Spuren 4, 8) zu beobachten ist, während im Falle des Kontrollproteins wt6HeGFP, dem die Zellpermeabilität vermittelnde Sequenz fehlt, dies weder nach 10 Min. (Spuren 1, 5) noch nach 20 Minuten (Spuren 3, 7) zu beobachten ist.

Diese Ergebnisse zeigen, dass DHBV-ZPP in der Lage ist, als Carrier für andere Proteine zu wirken.

## Patentansprüche

1. Zellpermeabilität-vermittelndes Polypeptid (ZPP), das eine Aminosäuresequenz, die ausgewählt ist aus der Gruppe bestehend aus umfasst und über kovalente oder nicht-kovalente Bindungen mit einem in eine Zelle zu transportierenden Molekül verbunden ist, mit der Maßgabe, dass das ZPP kein natives HBV Oberflächenprotein ist.

2. Nukleinsäuresequenz, kodierend für ein ZPP darstellendes Polypeptid, das als Fusionspolypeptid mit einer in eine Zelle zu transportierenden Substanz vorliegt und eine wie in Anspruch 1 angegebene Aminosäuresequenz umfasst.

3. Nukleinsäuresequenz nach Anspruch 2, wobei die Nukleinsäuresequenz eine DNA-Sequenz ist.

4. Nukleinsäuresequenz nach Anspruch 3, die mit der DNA-Sequenz CCC ATA TCG TCA ATC TTC TCG AGG ATT GGG GAC CCT bei 20°C unter dem Schmelzpunkt der DNA-Sequenz hybridisiert.

5. Expressionsvektor, umfassend eine Nukleinsäuresequenz nach einem der Ansprüche 2 bis 4.

6. Transformierte Zelle bzw. transformiertes Bakterium, enthaltend einen Expressionsvektor nach Anspruch 5.

7. Verwendung eines Zellpermeabilität-vermittelnden Polypeptids (ZPP) in vitro zur Vermittlung von Zellpermeabilität an eine in eine Zelle zu transportierende Substanz, wobei das ZPP eine Aminosäuresequenz, ausgewählt aus der Gruppe bestehend aus umfasst und über kovalente oder nicht-kovalente Bindungen mit der Substanz verbunden wird.

8. Verwendung nach Anspruch 7, wobei die Substanz aus Polypeptiden, Nukleinsäuren und chemischen Verbindungen ausgewählt ist.

9. Verwendung nach Anspruch 7 zur Herstellung eines Arzneistoffs.

## Claims

1. Cell permeability mediating polypeptide (CPP) comprising an amino acid sequence selected from the group consisting of and being linked via covalent or non-covalent bonds with a molecule which is to be transported into a cell, with the proviso that said CPP is not a native HBV surface protein.

2. Nucleic acid sequence encoding a polypeptide representing a CPP which polypeptide is present as fusion polypeptide with a substance which is to be transported into a cell and comprises an amino acid sequence set forth in claim 1.

3. Nucleic acid sequence according to claim 2 wherein the nucleic acid sequence is a DNA sequence.

4. Nucleic acid sequence according to claim 3 which hybridizes with the DNA sequence CCC ATA TCG TCA ATC TTC TCG AGG ATT GGG GAC CCT at 20°C below the melting point of said DNA sequence.

5. Expression vector comprising a nucleic acid sequence according to any one of claims 2 to 4.

6. Transformed cell or transformed bacterium comprising an expression vector according to claim 5.

7. Use of a cell permeability mediating polypeptide (CPP) in vitro for providing cell permeability to a substance which is to be transported into a cell, wherein said CPP comprises an amino acid sequence selected from the group consisting of and is linked with said substance via covalent or non-covalent bonds.

8. Use according to claim 7 wherein said substance is selected from polypeptides, nucleic acids and chemical compounds.

9. Use according to claim 7 for preparing a medicament.

## Revendications

1. Polypeptide de médiation de la perméabilité cellulaire (CPP), comprenant une séquence d'acides aminés choisie dans le groupe consistant en: et étant lié via des liaisons covalentes ou non-covalentes à une molécule qui est destinée à être transportée dans une cellule, avec pour condition que ledit CPP ne soit pas une protéine de surface de VHB native.

2. Séquence d'acide nucléique codant pour un polypeptide représentant un CPP, lequel CPP est présent sous forme de polypeptide de fusion avec une substance qui est destinée à être transportée dans une cellule et comprend une séquence d'acides aminés indiquée dans la revendication 1.

3. Séquence d'acide nucléique selon la revendication 2, dans laquelle la séquence d'acide nucléique est une séquence d'ADN.

4. Séquence d'acide nucléique selon la revendication 3, qui hybride avec la séquence d'ADN à 20°C en dessous du point de fusion de ladite séquence d'ADN.

5. Vecteur d'expression comprenant une séquence d'acide nucléique selon l'une quelconque des revendications 2 à 4.

6. Cellule transformée ou bactérie transformée, comprenant un vecteur d'expression selon la revendication 5.

7. Utilisation d'un polypeptide de médiation de la perméabilité cellulaire (CPP) in vitro pour procurer une perméabilité cellulaire à une substance qui est destinée à être transportée dans une cellule, tandis que ledit CPP comprend une séquence d'acides aminés choisie dans le groupe consistant en: et est lié avec ladite substance via des liaisons covalentes ou non-covalentes.

8. Utilisation selon la revendication 7, dans laquelle ladite substance est choisie parmi les polypeptides, les acides nucléiques et les composés chimiques.

9. Utilisation selon la revendication 7 pour la préparation d'un médicament.


### SEQUENZPROTOKOLL

<110> Eberhard Hildt, Stephanie Schmidt
<120> Zellpermeabilität-vermittelndes Polypeptid
<130> F 1704
<140> PCT/DE99/03506
   <141> 1999-11-03
<150> DE 198 50 718.6
   <151> 1998-11-03
<160> 20
<170> PatentIn Ver. 2.1
<210> 1
   <211> 36
   <212> DNA
   <213> Künstliche Sequenz
<220>
   <223> Beschreibung der künstlichen Sequenz: Zellpermeabilität-vermittelndes Polypeptid
<220>
   <221> CDS
   <222> (1)..(36)
<400> 1
   ccc tta tcg tca atc ttc tcg agg att ggg gac cct                36
   Pro Leu Ser Ser Ile Phe Ser Arg Ile Gly Asp Pro
     1               5                  10
<210> 2
   <211> 12
   <212> PRT
   <213> Künstliche Sequenz
<220>
   <223> Beschreibung der künstlichen Sequenz: Zellpermeabilität-vermittelndes Polypeptid
<400> 2
   Pro Leu Ser Ser Ile Phe Ser Arg Ile Gly Asp Pro
   1 5 10
<210> 3
   <211> 36
   <212> DNA
   <213> Hepadnavirus
<220>
   <221> CDS
   <222> (1)..(36)
<400> 3
   ccc tta tcg tca atc ttc tcg agg att ggg gac cct
   Pro Leu Ser Ser Ile Phe Ser Arg Ile Gly Asp Pro
   1 5 10
<210> 4
   <211> 12
   <212> PRT
   <213> Hepadnavirus
<400> 4
   Pro Leu Ser Ser Ile Phe Ser Arg Ile Gly Asp Pro
   1 5 10
<210> 5
   <211> 36
   <212> DNA
   <213> Hepadnavirus
<220>
   <221> CDS
   <222> (1)..(36)
<400> 5
   ccc ata tcg tca atc ttc tcg agg att ggg gac cct 36
   Pro Ile Ser Ser Ile Phe Ser Arg Ile Gly Asp Pro
   1 5 10
<210> 6
   <211> 12
   <212> PRT
   <213> Hepadnavirus
<400> 6
   Pro Ile Ser Ser Ile Phe Ser Arg Ile Gly Asp Pro
   1 5 10
<210> 7
   <211> 36
   <212> DNA
   <213> Hepadnavirus
<220>
   <221> CDS
   <222> (1)..(36)
<400> 7
   ccc ata tcg tca atc ttc tcg agg act ggg gac cct                36
   Pro Ile Ser Ser Ile Phe Ser Arg Thr Gly Asp Pro
     1               5                  10
<210> 8
   <211> 12
   <212> PRT
   <213> Hepadnavirus
<400> 8
   Pro Ile Ser Ser Ile Phe Ser Arg Thr Gly Asp Pro
     1               5                  10
<210> 9
   <211> 36
   <212> DNA
   <213> Hepadnavirus
<220>
   <221> CDS
   <222> (1)..(36)
<400> 9
   cac atc tcg tca atc tcc gcg agg act ggg gac cct                36
   His Ile Ser Ser Ile Ser Ala Arg Thr Gly Asp Pro
     1               5                  10
<210> 10
   <211> 12
   <212> PRT
   <213> Hepadnavirus
<400> 10
   His Ile Ser Ser Ile Ser Ala Arg Thr Gly Asp Pro
     1               5                  10
<210> 11
   <211> 12
   <212> PRT
   <213> Hepadnavirus
<400> 11
   Leu Leu Asn Gln Leu Ala Gly Arg Met Ile Pro Lys
   1 5 10
<210> 12
   <211> 12
   <212> PRT
   <213> Hepadnavirus
<400> 12
   Pro Leu Ser Ser Ile Phe Ser Arg Ile Gly Asp Pro
   1 5 10
<210> 13
   <211> 12
   <212> PRT
   <213> Hepadnavirus
<400> 13
   Thr Ile Asp His Val Leu Asp His Val Gln Thr Met
   1 5 10
<210> 14
   <211> 12
   <212> PRT
   <213> Hepadnavirus
<400> 14
   Pro Leu Ser Ser Ile Phe Ser Arg Ile Gly Asp Pro
   1 5 10
<210> 15
   <211> 12
   <212> PRT
   <213> Hepadnavirus
<400> 15
   Thr Ile Gln His Val Met Asp His Ile Asp Ser Val
   1 5 10
<210> 16
   <211> 12
   <212> PRT
   <213> Hepadnavirus
<400> 16
   Pro Leu Ser Ser Ile Phe Ser Arg Ile Gly Asp Pro
   1 5 10
<210> 17
   <211> 12
   <212> PRT
   <213> Hepadnavirus
<400> 17
   Thr Leu Ser Pro Val Val Pro Thr Val Ser Thr Ile
   1 5 10
<210> 18
   <211> 12
   <212> PRT
   <213> Hepadnavirus
<400> 18
   Pro Leu Ser Ser Ile Phe Ser Arg Ile Gly Asp Pro
   1 5 10
<210> 19
   <211> 12
   <212> PRT
   <213> Hepadnavirus
<400> 19
   Thr Leu Ser Pro Val Val Pro Thr Val Ser Thr Thr
   1 5 10
<210> 20
   <211> 12
   <212> PRT
   <213> Hepadnavirus
<400> 20
   Pro Leu Ser Ser Ile Phe Ser Arg Ile Gly Asp Pro
   1 5 10